# EUROPEAN PATENT SPECIFICATION

(11) **EP 0 416 842 B1**
(45) Date of publication and mention of the grant of the patent: **06.12.1995**
(21) Application number: 90309608.9
(22) Date of filing: 03.09.1990
(51) Int. Cl.: A61L 15/44, A61L 15/58

(54) **Solid matrix system for transdermal drug delivery**
Festes Matrixsystem zur transdermalen Arzneimittelfreisetzung
Système de matrice solide pour l'administration transcutanée de médicaments

(30) Priority: 08.09.1989 US 405630
(43) Date of publication of application: 13.03.1991
(73) Proprietor: CYGNUS, INC., Redwood City, CA 94063 (US)
(72) Inventor: Chiang, Chia-Ming, Foster City, CA 94404 (US); Tenzel, Renee Ann, Mountain View, CA 94043 (US)
(74) Representative: Goldin, Douglas Michael

(56) References cited:
- EP-A- 0 156 080
- EP-A- 0 209 975
- EP-A- 0 223 524
- EP-A- 0 252 712
- EP-A- 0 328 806
- WO-A-86/00814
- GB-A- 2 086 224

## Description

This invention relates generally to the transdermal and transmucosal administration of drugs. More particularly, it concerns a configuration for transdermal or transmucosal steroid drug delivery devices which enables the administration of effective levels of drugs without the necessity for coadministration with skin penetration rate enhancers.

Transdermal delivery of drugs, that is, delivery of drugs through the skin, provides many advantages. The method is a comfortable, convenient, and noninvasive way of administering drugs. Many of the variables and side effects associated with oral administration are eliminated. Since the early 1970s, there has been substantial effort spent on developing particular systems for effectively delivering drugs in a transdermal mode. A variety of devices containing, at minimum, a drug reservoir and a backing, and optionally containing other layers, such as an adhesive layer for adhering the device to the patient and a drug release rate controlling layer for moderating delivery rate have been constructed. With certain drugs, in particular scopolamine and nitroglycerine, it is feasible to construct a transdermal drug delivery device which will achieve therapeutically effective levels of the drug in the patient. Commercial products have been introduced to deliver these two materials. However, one of the key problems with transdermal administration of many other drugs has been the low penetration or permeation rate of drug through the skin of the patient. The research over the past two decades has identified various skin permeation enhancers. These materials increase the rate of penetration of drugs across the skin.

Typical enhancers in the art include ethanol, glycerol monolaurate, PGML (polyethylene glycol monolaurate) and dimethylsulfoxide. Many highly attractive drugs, such as estradiol and progestins, are commonly formulated with enhancers for transdermal delivery.

The use of permeation enhancers is not without its drawbacks. For one, the permeation enhancer typically is co-administered with the desired drug. That is, the permeation enhancer passes through the patient's skin at the same time the drug does. Depending upon the exact nature of the permeation enhancer, this can lead to side effects related directly to the permeation enhancers.

Another disadvantage is that the enhancers are often organic solvents, which can in some cases react with and alter the character of the drug being delivered. In addition, the enhancers can interact with the patient's skin, in some cases for example causing irritation. Moreover, enhancers can interfere with the mechanical properties of the devices, such as interfering with the effectiveness of adhesive layers.

EP-A-156,080 discloses a transdermal drug delivery system in which the drug may be a steroid. The steroid is present at a concentration of greater than its saturated solubility.

EP-A-223,524 discloses a dermal bandage which may contain a wide variety of drugs dispersed in a mixture of at least one carboxylic acid and/or polycarboxylic acid anhydride, and a vinyl acetate polymer.

The present invention seeks to provide drug-matrix constructions which, when contacted with patient skin, allow high rates of delivery of steroid drugs without the necessity of added permeation enhancers previously required to reach therapeutic blood levels.

It has now been found that the necessity for incorporating permeation enhancers into steroid drug delivery systems can be eliminated by incorporating the drug in a matrix comprising a particular vinyl acetate-acrylate copolymer and delivering the drug to the patient from this matrix.

The transdermal or transmucosal drug delivery device is used to administer the drug to a predetermined area of skin or mucosa of a patient.

The present invention provides a drug-containing matrix for use in a transdermal or transmucosal drug delivery device for administering at least one steroid drug to an area of skin or mucosa comprising the drug dispersed in an amount below or at saturation in a body of a pressure sensitive adhesive acrylate copolymer, said acrylate copolymer comprising a copolymer of at least 2-ethylhexyl acrylate and vinyl acetate, said matrix being essentially free of a skin permeation enhancer.

The present invention also provides a transdermal or transmucosal drug delivery device for administering at least one steroid drug to an area of skin or mucosa comprising
(a) a layer of backing material which is substantially impermeable to the drug and laminated thereto; and
(b) an adhesive drug-containing matrix as defined above.

The present invention further provides a device as defined above for use in a method of delivering a drug to a patient by applying the matrix of the device to the skin or mucosa of said patient.

The device of the present invention can give rise to high rates of drug delivery with no added permeation enhancer present.

In preferred embodiments, the matrix can additionally contain hydrophilic polymers such as water-soluble polymers, for example polyvinyl alcohol or polyvinyl pyrrolidone.

This invention will be described with reference being made to the accompanying drawings, in which:
FIG. 1 is a not-to-scale cross-sectional view of one form of drug delivery device constructed using the teachings of this invention;
FIG. 2 is a not-to-scale cross-sectional view of a second form of drug delivery device constructed using the teachings of this invention.
FIG. 3 is a graph illustrating the fluxes attainable in drug delivery systems employing the present invention.

In this specification certain terms will be used which have defined meanings.

By "transdermal" delivery is intended both transdermal (or "percutaneous") and transmucosal administration, i.e. delivery by passage of a drug through the skin or mucosal tissue and into the bloodstream.

"Carriers" or "vehicles" as used herein refer to carrier materials suitable for transdermal or transmucosal drug administration, and include any such materials known in the art, for example any liquid, gel, solvent, liquid diluent or solubilizer which is nontoxic and which does not interact with other components of the composition or the skin in a deleterious manner. Examples of suitable carriers for use herein include water, mineral oil, silicone, liquid sugars, waxes, petroleum jelly, and a variety of other oils and polymeric materials. In addition, one or both of the components of the present enhancer composition may also serve as a carrier.

"Permeation enhancement" and "permeation enhancers" as used herein relate to the process and added materials which bring about an increase in the permeability of skin to a poorly skin permeating pharmacologically active agent, i.e., so as to increase the rate at which the drug permeates through the skin and enters the bloodstream. The enhanced permeation effected through the use of such enhancers is not required when the matrix compositions of the present invention are employed.

By the term "pharmacologically active agent" or "drug" as used herein is meant a steroid suitable for transdermal or transmucosal administration which induces a desired systemic effect.

Steroid drugs such as hormones are the drugs for use in conjunction with the drug delivery device and matrix composition of the present invention. Steroid drugs have been difficult materials to administer transdermally, historically because of their generally poor (low) skin permeation properties. Historically, skin permeation enhancers have been used to achieve therapeutic blood levels in patients. Examples of steroid drugs useful herein include: progestogens such as norethindrone, norethindrone acetate, desogestrel, 3-keto desogestrel, gestadene and levonorgestrel; estrogens such as progesterone and estradiol and its esters, for example estradiol valerate, cyprionate, decanoate and acetate, as well as ethinyl estradiol; androgens such as testosterone and its esters; and corticosteroids such as cortisone, hydrocortisone, and fluocinolone acetonide. In a particularly preferred embodiment, the devices and matrices of the invention include one or more estrogens, in particular estradiol, and may include one or more progestogens as well.

By a "therapeutically effective" amount of a pharmacologically active agent is meant a nontoxic but sufficient amount of a compound to provide the desired therapeutic effect.

"Water-soluble polymer" means a hydrophilic polymer having a solubility in water of greater than 0.1% by weight.

A key element of this invention is the use of a matrix which permits high delivery rates for steroid drugs without the use of added skin penetration rate enhancers.

It has been found that matrices made from particular acetate-acrylate copolymers give rise to unexpectedly high rates of drug delivery. These acetate-acrylate copolymer materials are available commercially. For example, Monsanto Chemical Company distributes a family of vinyl acetate-acrylate copolymer resin solutions under the trademarks GELVA® 737 (approximately 72 wt% 2-ethylhexyl acrylate and approximately 28 wt% vinyl acetate) and GELVA® 788 (approximately 70 wt% 2-ethylhexyl acrylate and approximately 30 wt% vinyl acetate) and Morton Thiokol, Inc. distributes acrylate copolymers under the trademarks Morstik 207A and Morstik 607 (approximately 85 wt% 2-ethylhexyl acrylate, approximately 10 wt% methyl acrylate, approximately 3 wt% acrylic acid and approximately 2 wt% vinyl acetate).

These acrylate copolymer materials can be used separately or in mixtures. Several specific materials which have given rise to superior results are the Morstik 607 material, the GELVA® materials, and mixtures of from about 20:1 to about 1:1 parts GELVA® 737 and GELVA® 788 (ratios given as weight ratios of GELVA® 737 to GELVA® 788). All of these materials are solvent based but form films following casting and removal of the solvent. The term "solid" is used broadly since the "solid" product is generally a tacky, amorphous (i.e. pressure sensitive adhesive) non-flowing material.

These materials are typically available as solutions in organic solvents such as toluene, ethanol, isopropanol or ethyl acetate. These solvents are substantially eliminated from the matrix during fabrication.

These copolymers have the property of being high tack pressure sensitive adhesive when dried and/or cured. Thus, the matrices formed from these materials can adhere directly to the patient's skin without the need for additional separate adhesives.

The devices of this invention include a solid body of the matrix-forming copolymer material throughout which the drug is incorporated. This incorporation can be carried out by simply dissolving or otherwise finely dispersing the drug in a solution of the matrix material to yield a solution or slurry, casting the slurry or solution that contains the drug matrix and then evaporating the volatile solvents to give a solid matrix with drug incorporated therein.

The incorporating can be carried out with conventional polymer solution-handling equipment such as mixers or mills, and can be completed in from a few seconds to a few hours, depending upon mixing conditions. Generally, it is continued until a uniform solution or homogeneous dispersion is attained.

Although not known with certainty and without intention to be bound to any particular mode of operation, it is believed that the high drug flux rate obtained using the matrices of this invention may in part result from the fact that the drug is contacted with the matrix-forming solution prior to solidification. This contact may result in at least partially dissolving the drug in the matrix phase, changing the drug's crystalline form to a more polymorphic structure, or forming a microdispersion of the drug in the matrix polymer.

The casting can be carried out using, for example, manual casting machines or doctor blades or can be carried out with commercial film casting equipment for large scale production.

The thickness of the matrix can vary from 10 micrometers to about 250 micrometers. Preferred thicknesses are from 15 to 100 micrometers. These relatively thin layer thicknesses are of advantage in assuring the completeness of the subsequent solvent removal step.

The solvent removal should be thorough and is carried out using heat, air flow and/or a vacuum. Temperatures should be held below temperatures at which significant degradation of drug occurs and typically range from room temperature (approximately 20°C to 25°C) to about 100°C although higher temperatures can be used if the nature of the drug permits.

The solvent removal should be carried out completely until substantially no solvent remains, for example until the solvent level is less than 5%, preferably less than 1%, by weight.

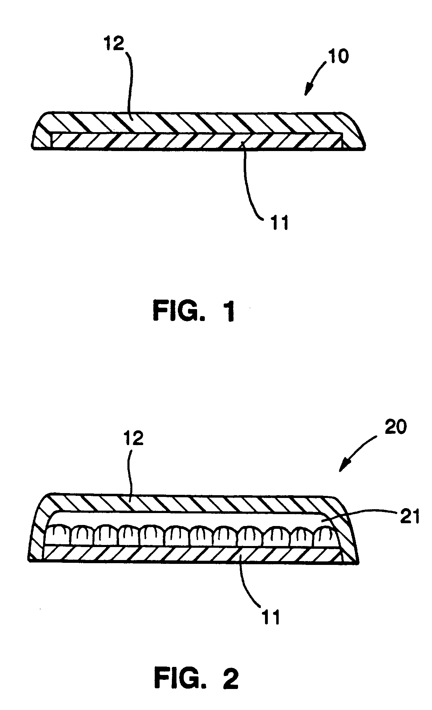

As shown in Figure 1, the device 10 of this invention includes a matrix 11 having drug dispersed therethrough and can in addition include a backing layer 12. The backing 12 is provided to contain the drug and prevent its loss.

The matrices and devices of this invention can be of any size suitable for transdermal drug delivery. This encompasses an area of from about 0.5 cm² to about 100 cm².

The backing 12 is generally a water-occlusive layer, preferably made of a sheet or film of a preferably flexible elastomeric material that is substantially impermeable to the selected drug. The layer is preferably of the order of 1 micrometer to 100 micrometers in thickness, and may or may not contain pigment. The layer is preferably of a material that permits the device to mimic the contours of the skin and be worn comfortably on areas of skin, such as at joints or other points of flexure, that are normally subjected to mechanical strain with little or no likelihood of the device disengaging from the skin due to differences in the flexibility or resiliency of the skin and the device. Elastomeric materials generally present these desired properties. Examples of elastomeric polymers that are useful for making layer 11 are polyether block amide copolymers (e.g., PEBAX copolymers), polyethylene methyl methacrylate block copolymers (EMA) such as NUKRELL polymers, polyurethanes such as PELLATHANE or ESTANE polymers, silicone elastomers, polyester block copolymers that are composed of hard and soft segments (e.g., HYTREL polymers), rubber-based polyisobutylene, styrene, and styrene-butadiene and styrene-isoprene copolymers. Polymers that are flexible but not elastomeric include polyethylene, polypropylene, polyesters, e.g., polyester terephthalate (PET), which may be in the form of films or laminates. The preferred polymer used for the backing will depend on the material or drug incorporated into the device and on the nature of, for example, any vehicles or solubilizers that are used.

In a second embodiment, as shown in Figure 2, a device 20 can include in addition to the matrix 11 and backing 12 as just set forth a drug reservoir 21. This reservoir can be a void in which additional drug and (as needed) carrier are lodged or can contain a porous substrate such as a porous polymer or sponge which holds and easily delivers drug to the matrix 11 for continuous administration to the patient.

As previously pointed out, the devices of the invention can advantageously contain added water-soluble water-absorptive polymer. These materials are added solely to improve long-term wearing properties by absorbing moisture from the wearer's skin and are not seen to modify or enhance the rate of drug delivery.

The water-soluble polymers that can be used in the invention include, for example, polyvinyl alcohol, gelatine, polyacrylic acid, sodium polyacrylate, methylcellulose, carboxymethylcellulose, polyvinylpyrrolidone, gum acacia, gum tragacanth, carrageenan, gum guar and the like gums and dextrans. They also include suitable cross-linked reaction products of these materials which may offer improved cohesion. These water-soluble polymers can be used either singly or in combinations of two or more. These water-soluble polymers can be of molecular weights varying from as low as 10,000 to several million (3,000,000). Polyvinyl alcohol and polyvinyl pyrollidone, two preferred polymers, are commercially available in sizes throughout the range.

The devices of this invention have a matrix composed of acrylate copolymer and drug. The matrix may also include a water-soluble polymer.

The matrix may, in addition, include one or more selected carriers or excipients, and various agents and ingredients commonly employed in dermatological ointments and lotions. For examples, fragrances, opacifiers, preservatives, antioxidants, gelling agents, perfumes, thickening agents, stabilisers, surfactants, emollients and colouring agents may be present.

The relative amounts of the components in these compositions can vary a great deal. For example, the amount of drug or drugs present in the composition will depend on a variety of factors, including the disease to be treated, the nature and activity of the drug, the desired effect, possible adverse reactions, the ability and speed of the drug to reach its intended target, and other factors within the particular knowledge of the patient and physician.

In typical embodiments, the matrix will contain from about 0.5% up to about 25% by weight (based on the total matrix weight) drug; for example, 1 to 10% by weight estrogen (estradiol) and 1 to 15% by weight progestogen, (norethindrone acetate) will be present in a preferred post-menopausal syndrome or contraceptive patch, and 1 to 15% by weight estradiol will be present in a preferred patch releasing only estrogen.

The matrix may be formulated so that the selected drug is contained therein below saturation or at saturation.

The amount of water-soluble polymer may range from 0% (in light of its optional character) to as much as 40% by weight. When water-soluble polymer is present, use levels of 2 to 30% by weight are preferred.

The present invention is now further described in the following Examples.

### Examples

### Example 1

Monolith matrix systems in accord with this invention and based on estradiol, norethindrone, norethindrone acetate, and levonorgesterol were prepared by the following procedures: The drug was mixed and sonicated with or without a known skin penetration enhancer (PGML) for 10 minutes. Typical polymer solvents, if present, included lower alcohols such as ethanol and isopropanol and lower alkanoic acid esters such as ethylacetate. (These solvent materials were later removed during drying.) Monsanto GELVA® 737 vinyl acetate acrylate copolymer resin pressure-sensitive adhesive solution was added to the drug-solvent mixture and rotated overnight. The drug-solvent-polymer mixture was then cast to about 100 micrometers thickness on a polyester film (#1022 release liner). The solvent in the polymer system was evaporated in a 75°C forced air oven for 15 to 20 minutes. The resultant drug reservoir matrix was laminated with another polyester film (3M #1022). For comparison, similar compositions (with and without PGML) were prepared using Dow Corning silicone as the matrix polymer.

Modified Franz flow-through cells were used for in vitro penetration studies which were carried out to determine the efficiency of the present matrices at delivering drugs. One of the two polyester layers was peeled off of the drug matrix layer. The drug matrix layer was gently pressed onto the stratum corneum of human cadaver skin membrane. This skin membrane with the backing and matrix affixed thereto was then mounted between the two half-cells and fastened with a clamp. The receiver compartment was filled with 0.1% gentamycin in distilled, deionized water and the temperature was maintained at 32°C. Samples were taken at preset intervals and assayed by HPLC. The flux was calculated from the slope of the cumulative amounts of the drug in the receiver compartment versus time.

The fluxes of estradiol, norethindrone, norethindrone acetate and levonorgesterol through human cadaver skin are summarized in Table 1. The fluxes for all of the drugs tested (i.e., estradiol, norethindrone acetate, norethindrone and levonorgesterol) were not affected by incorporation of the enhancer (PGML) in the acrylate matrix. However, the fluxes of norethindrone and norethindrone acetate did increase significantly when PGML was used in the silicone matrix. More importantly, the fluxes of estradiol, norethindrone and levonorgesterol from acrylate copolymer systems without enhancers were all comparable to those with PGML in the silicon matrix system. Although the flux of norethindrone acetate from the acrylate copolymer matrix was low, it may be due to a higher solubility of norethindrone acetate in the acrylate copolymer system. Therefore, the low loading (1%) of norethindrone acetate may not have enabled a higher driving force for the diffusion. By this reasoning, the norethindrone flux could be increased by increasing the drug loading in the polymer matrix until maximum thermodynamic activity is reached. It is an advantage of the present invention that since no enhancer is present, higher thermodynamic activity can be achieved with less drug.

**Table 1**

| In Vitro Skin Fluxes of Estradiol and Progestogens Through Human Cadaver Skin From Polymer Matrix With or Without PGML | | |
|---|---|---|
| Drug | System | Fluxes (»g/cm²h) |
| Estradiol (E2) | E2/PGML/silicone (5:14:81, w/w) | 0.14±0.05 |
| | E2/PGML/acrylate (1:14:85) | 0.17±0.00 |
| | E2/acrylate (1:99) | 0.12±0.00 |
| Norethindrone (N) | N/PGML/silicone (1:14:85, w/w) | 0.20±0.02 |
| | N/silicone (1:99) | 0.07±0.02 |
| | N/PGML/acrylate (1:14:85) | 0.26±0.08 |
| | N/acrylate (1:99) | 0.24±0.09 |
| Norethindrone acetate (NA) | NA/PGML/silicone (1:14:85, w/w) | 0.54±0.10 |
| | NA/silicone (1:99) | 0.17±0.00 |
| | NA/PGML/acrylate (1:14:85) | 0.06±0.03 |
| | NA/acrylate | 0.05±0.01 |
| Levonorgesterol (LG) | LG/PGML/silicone (1:14:85, w/w) | 0.09±0.02 |
| | LG/PGML/acrylate (1:14:85) | 0.22±0.02 |
| | LG/acrylate (1:99) | 0.17±0.04 |

### Example 2

A series of monolith systems of norethindrone acetate was prepared by the following procedures. Norethindrone acetate was mixed and sonicated with solvents and with or without enhancers (PGML and dipropylene glycol monoethyl ether "Transcutanol"(TC)) for 10 minutes. A solution of acrylate copolymer pressure-sensitive adhesive was added to the drug-vehicle mixture and rotated overnight.

The drug-solvent-polymer mixture was then cast on a polyester film (3M #1022 release liner). The solvent in the polymer system was thoroughly evaporated in a 75°C forced air oven for 15-20 minutes. The resultant drug reservoir polymer matrix was laminated with another polyester film (3M #1022).

The in vitro permeation studies and data analyses were the same as in Example 1. Again, the fluxes of norethindrone acetate without enhancers were comparable to those with enhancers in the polymer matrix (Table 2). The results also show that in the acrylate matrix, the fluxes of norethindrone acetate were essentially independent of the presence of enhancers. Acrylate matrices without enhancers gave fluxes which are comparable to fluxes possible in conventional silicone matrices only with added enhancers.

**Table 2**

| In Vitro Skin Fluxes of Norethindrone Acetate From Polymer Matrix With and Without Enhancers | |
|---|---|
| System | Fluxes (»g/cm²/hr) |
| NA/PGML/silicone (2/10/88, w/w) | 0.19±0.03 |
| NA/TC:PGML/acrylate (4/10/86, w/w) | 0.18±0.02 |
| NA/PGML/acrylate (4/10/86, w/w) | 0.14±0.02 |
| NA/acrylate (4/96, w/w) | 0.15±0.03 |

### Example 3

A series of prototype systems was made. The drug reservoir layers were prepared as described in Example 2. However, 20% PVP (BASF, K-30) was suspended with the drugs (E2 and NA) in the polymer solution (Morstik 607) with addition of isopropanol. The drugs, PVP, polymer and solvents were then rotated overnight and a homogeneous solution was obtained. A uniform drug reservoir matrix was then cast on a polyester film (3M #1022). The solvent in the system was evaporated in a 75°C forced air oven for 30 minutes. After cooling, the reservoir layer was laminated with a layer of polyisobutylene to an elastomeric backing membrane (Bertek #438, #810, or 3M 166) or laminated directly to a second layer of 3M #1022 polyester.

The in vitro permeation studies and data analyses were the same as in Example 1. The results show that in acrylate systems, the fluxes of both estradiol and norethindrone acetate were high without employing enhancers (Table 3). The fluxes of the drugs increased as the percentage of drug increased in these systems. The fluxes reached maximum when 4% estradiol and 10% norethindrone acetate were present in the drug matrices. The flux of 2% estradiol in the system was 0.25 »g/cm²/h, which is comparable to the commercial estrogen patches which include permeation enhancers. The flux of 10% norethindrone acetate reached 0.76 »g/cm²/h, which indicates that effective levels of progestogens can be delivered from the system as well. The fluxes were comparable whether the backing material used was an occlusive polyester film or combined layers of PIB and an occlusive elastomeric layer.

**Table 3**

| In Vitro Skin Fluxes of Estradiol and Norethindrone Acetate from Acrylate Matrix Systems with Polyvinylpyrrolidone Incorporated | | | |
|---|---|---|---|
| System | | Fluxes (»g/cm²/h) | |
| Drug Reservoir | Backing Layer | Estradiol | Norethindrone |
| E2/NA/PVP/acrylate (2/5/20/73, w/w) | Polyester | 0.25±0.03 | 0.38±0.06 |
| E2/NA/PVP/acrylate (2/5/20/73, w/w) | PIB & 3M 166 | 0.28±0.04 | 0.40±0.04 |
| E2/NA/PVP/acrylate (2/5/20/73, w/w) | PIB & Bertek | 428 0.27±0.01 | 0.39±0.00 |
| E2/NA/PVP/acrylate (4/10/20/66, w/w) | Polyester | 0.50±0.13 | 0.76±0.17 |
| E2/NA/PVP/acrylate (4/10/20/66, w/w) | PIB & 3M 166 | 0.43±0.05 | 0.64±0.05 |
| E2/NA/PVP/acrylate (4/10/20/66, w/w) | PIB & Bertek | 810 0.39±0.07 | 0.58±0.11 |

### Example 4

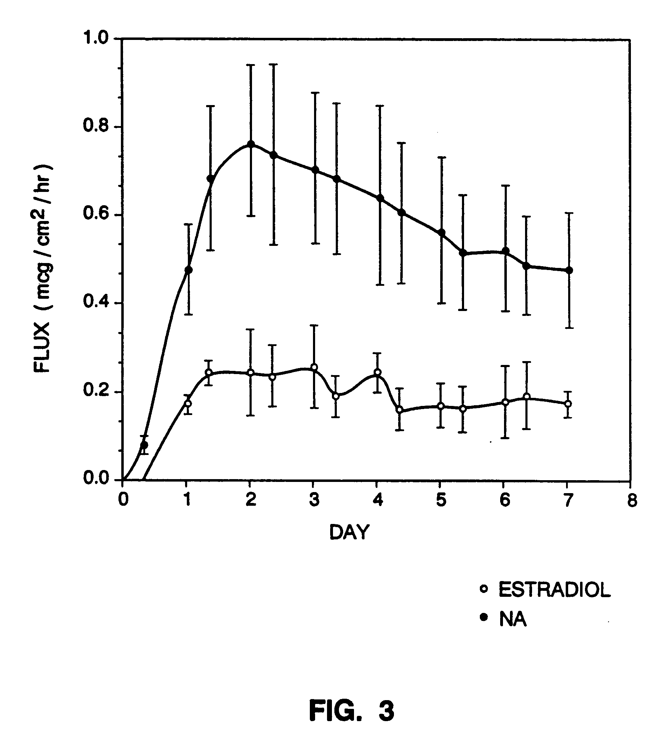

In vitro Franz flow-through cells were used to determine the penetration of mixtures of norethindrone acetate and estradiol in an acrylate matrix system. The system was similar to Example 3. The drug reservoir layer was made with 2% estradiol, 10% norethindrone acetate and 20% PVP (BASF, K-30) in Morstik acrylate pressure-sensitive adhesive (#607). The backing layer contained an occlusive PIB layer and an elastomeric layer (Bertek 810). The in vitro skin fluxes for both drugs during a 7 day period are presented in FIG. 3. This figure shows that the flux of both drugs reached steady state within 24 hours and then maintained at steady state for the rest of the seven day permeation study. The average flux for estradiol was 0.21±0.06 (»g/cm²/h) while the average flux for norethindrone acetate was 0.62±0.16 (»g/cm²/h). These values suggest that sufficient amounts of estradiol and norethindrone acetate can be delivered without incorporation of an enhancer.

### Examples 5-8

The experiments of Example 4 are repeated making changes in the composition of the reservoir:

In Example 5, the Morstik 607 is replaced with Monsanto GELVA® 737.

In Example 6, the Morstik 607 is replaced with a 4:1 mixture of GELVA® 737:GELVA® 788.

In Example 7, the Morstik 607 is replaced with a 9:1 mixture of GELVA® 737:GELVA® 788.

In Example 8, the PVP is replaced with similar levels of polyvinylalcohol.

In each case favorable results similar to those seen in Example 4 are attained.

## Claims (Claims for the following Contracting State(s): AT, BE, CH, DE, DK, FR, GB, IT, LI, LU, NL, SE)

1. A drug-containing matrix for use in a transdermal or transmucosal drug delivery device for administering at least one steroid drug to an area of skin or mucosa comprising the drug dispersed in an amount below or at saturation in a body of a pressure sensitive adhesive acrylate copolymer, said acrylate copolymer comprising a copolymer of at least 2-ethylhexyl acrylate and vinyl acetate, said matrix being essentially free of a skin permeation enhancer.

2. The matrix of claim 1 wherein the copolymer comprises approximately 72 wt% 2-ethylhexyl acrylate and approximately 28 wt% vinyl acetate (Monsanto GELVA® 737).

3. The matrix of claim 1 wherein the copolymer comprises approximately 70 wt% 2-ethylhexyl acrylate and approximately 30 wt% vinyl acetate (Monsanto GELVA® 788).

4. The matrix of claim 1 wherein the copolymer comprises approximately 85 wt% 2-ethylhexyl acrylate, approximately 10 wt% methyl acrylate, approximately 3 wt% acrylic acid and approximately 2 wt% vinyl acetate (Morton Thiokol Morstik 607).

5. The matrix of any one of claims 1 to 4 wherein the drug comprises norethindrone or norethindrone acetate.

6. The matrix of any one of claims 1 to 4 wherein the drug comprises a mixture of estrogen and progestogen.

7. A transdermal or transmucosal drug delivery device for administering at least one steroid drug to an area of skin or mucosa comprising
(a) a layer of backing material which is substantially impermeable to the drug and laminated thereto; and
(b) an adhesive drug-containing matrix as defined in any one of claims 1 to 6.

8. A device according to claim 7 for use in a method of delivering a drug to a patient by applying the matrix of the device to the skin or mucosa of said patient.

## Claims (Claims for the following Contracting State(s): ES, GR)

1. A process for producing a drug-containing matrix for use in a transdermal or transmucosal drug delivery device for administering at least one steroid drug to an area of skin or mucosa, which process comprises dispersing the drug in an amount below or at saturation in a body of a pressure sensitive adhesive acrylate copolymer, said acrylate copolymer comprising a copolymer of at least 2-ethylhexyl acrylate and vinyl acetate, said matrix being essentially free of a skin permeation enhancer.

2. The process of claim 1 wherein the copolymer comprises approximately 72 wt% 2-ethylhexyl acrylate and approximately 28 wt% vinyl acetate (Monsanto GELVA ® 737).

3. The process of claim 1 wherein the copolymer comprises approximately 70 wt% 2-ethylhexyl acrylate and approximately 30 wt% vinyl acetate (Monsanto GELVA ® 788).

4. The process of claim 1 wherein the copolymer comprises approximately 85 wt% 2-ethylhexyl acrylate, approximately 10 wt% methyl acrylate, approximately 3 wt% acrylic acid and approximately 2 wt% vinyl acetate (Morton Thiokol Morstik 607).

5. The process of any one of claims 1 to 4 wherein the drug comprises norethindrone or norethindrone acetate.

6. The process of any one of claims 1 to 4 wherein the drug comprises a mixture of estrogen and progestogen.

7. A process for producing a transdermal or transmucosal drug delivery device for administering at least one steroid drug to an area of skin or mucosa, which process comprises laminating:
(a) a layer of backing material which is substantially impermeable to the drug; and
(b) an adhesive drug-containing matrix obtainable by a process as defined in any one of claims 1 to 6.

## Patentansprüche (Patentansprüche für folgende(n) Vertragsstaat(en): AT, BE, CH, DE, DK, FR, GB, IT, LI, LU, NL, SE)

1. Arzneistoffhaltige Matrix zur Verwendung in einer perkutanen oder transmukosalen Arzneistoff-Freisetzungsvorrichtung zur Verabreichung mindestens eines Steroid-Arzneistoffes an eine Fläche der Haut oder Schleimhaut, umfassend den in einer Menge unterhalb der oder bei der Sättigung in einem Körper aus einem druckempfindlichen adhäsiven Acrylatcopolymeren dispergierten Arzneistoff wobei das Acrylatcopolymer ein Copolymer aus mindestens 2-Ethylhexylacrylat und Vinylacetat umfaßt, wobei die Matrix im wesentlichen keinen Hautdurchdringungsverstärker enthält.

2. Matrix nach Anspruch 1, wobei das Copolymer etwa 72 Gew.-% 2-Ethylhexylacrylat und etwa 28 Gew.-% Vinylacetat (Monsanto Gelva® 737) umfaßt.

3. Matrix nach Anspruch 1, wobei das Copolymer etwa 70 Gew.-% 2-Ethylhexylacrylat und etwa 30 Gew.-% Vinylacetat (Monsanto Gelva® 788) umfaßt.

4. Matrix nach Anspruch 1, wobei das Copolymer etwa 85 Gew.-% 2-Ethylhexylacrylat, etwa 10 Gew.-% Methylacrylat, etwa 3 Gew.-% Acrylsäure und etwa 2 Gew.-% Vinylacetat (Morton Thiokol/Morstik 607) umfaßt.

5. Matrix nach mindestens einem der Ansprüche 1 bis 4, wobei der Arzneistoff Norethindron oder Norethindronacetat umfaßt.

6. Matrix nach mindestens einem der Ansprüche 1 bis 4, wobei der Arzneistoff eine Mischung aus Östrogen und Progestogen umfaßt.

7. Perkutane oder transmukosale Armeistoff-Freisetzungvorrichtung zur Verabreichung mindestens eines Steroid-Arzneistoffes an eine Fläche der Haut oder Schleimhaut, umfassend:
(a) eine Rückenseitenmaterialschicht, welche im wesentlichen für den Arzneistoff undurchlässig und daran laminiert ist; und
(b) eine adhäsive arzneistoffhaltige Matrix nach einem der Ansprüche 1 bis 6.

8. Vorrichtung nach Anspruch 7 zur Verwendung bei einem Verfahren der Freisetzung eines Arzneistoffes an einen Patienten, indem die Matrix der Vorrichtung auf die Haut oder Schleimhaut des Patienten appliziert wird.

## Patentansprüche (Patentansprüche für folgende(n) Vertragsstaat(en): ES, GR)

1. Verfahren zur Herstellung einer arzneistoffhaltigen Matrix zur Verwendung in einer perkutanen oder transmukosalen Armeistoff-Freisetungsvorrichtung zur Verabreichung mindestens eines Steroid-Arzneistoffes an eine Fläche der Haut oder Schleimhaut, umfassend den in einer Menge unterhalb der oder bei der Sättigung in einem Körper aus einem druckempfindlichen adhäsiven Acrylatcopolymeren dispergierten Arzneistoff, wobei das Acrylatcopolymer ein Copolymer aus mindestens 2-Ethythexylacrylat und Vinylacetat umfaßt, wobei die Matrix im wesentlichen keinen Hautdurchdringungsverstärker enthält.

2. Verfahren nach Anspruch 1, wobei das Copolymer etwa 72 Gew.-% 2-Ethylhexylacrylat und etwa 28 Gew.-% Vinylacetat (Monsanto Gelva® 737) umfaßt.

3. Verfahren nach Anspruch 1, wobei das Copolymer etwa 70 Gew.-% 2-Ethythexylacrylat und etwa 30 Gew.-% Vinylacetat (Monsanto Gelva® 788) umfaßt.

4. Verfahren nach Anspruch 1, wobei das Copolymer etwa 85 Gew.-% 2-Ethylhexylacrylat, etwa 10 Gew.-% Methylacrylat, etwa 3 Gew.-% Acrylsäure und etwa 2 Gew.-% Vinylacetat (Morton Thiokol/Morstik 607) umfaßt.

5. Verfahren nach mindestens einem der Ansprüche 1 bis 4, wobei der Arzneistoff Norethindron oder Norethindronacetat umfaßt.

6. Verfahren nach mindestens einem der Ansprüche 1 bis 4, wobei der Arzneistoff eine Mischung aus Östrogen und Progestogen umfaßt.

7. Verfahren zur Herstellung einer perkutanen oder transmukosalen Arzneistoff-Freisetzungsvorrichtung zur Verabreichung mindestens eines Steroid-Arzneistoffes an eine Fläche der Haut oder Schleimhaut, umfassend das Laminieren:
(a) einer Rückenseitenmaterialschicht, welche im wesentlichen für den Arzneistoff undurchlässig ist; und
(b) einer adhäsiven arzneistoffhaltigen Matrix, die durch ein Verfahren nach einem der Ansprüche 1 bis 6 erhältlich ist.

## Revendications (Revendications pour l'(les) Etat(s) contractant(s) suivant(s): AT, BE, CH, DE, DK, FR, GB, IT, LI, LU, NL, SE)

1. Matrice contenant un médicament pour l'utilisation dans un dispositif de délivrance transdermique ou transmuqueux d'un médicament pour l'administration d'au moins un médicament stéroïdien à une zone de peau ou de muqueuse, comprenant le médicament dispersé en une proportion inférieure ou égale à la saturation dans un corps fait d'un copolymère d'acrylate adhésif sensible à la pression, ledit copolymère d'acrylate comprenant un copolymère d'au moins l'acrylate de 2-éthylhexyle et l'acétate de vinyle, ladite matrice étant essentiellement dépourvue d'un agent améliorant la penétration cutanée.

2. Matrice selon la revendication 1, ou le copolymère comprenant approximativement 72 % en poids d'acrylate de 2-éthylhexyle et approximativement 28 % en poids d'acétate de vinyle (Monsanto GELVA® 737).

3. Matrice selon la revendication 1, où le copolymère comprend approximatrvement 70 % en poids d'acrylate de 2-éthylhexyle et approximativement 30 % en poids d'acétate de vinyle (Monsanto GELVA® 788).

4. Matrice selon la revendication 1, où le copolymère comprend approximativement 85 % en poids d'acrylate de 2-éthylhexyle, approximativement 10 % en poids d'acrylate de méthyle, approximativement 3 % en poids d'acide acrylique et approximativement 2 % en poids d'acétate de vinyle (Morton Thiokol Morstik 607).

5. Matrice selon l'une quelconque des revendications 1 à 4, où le médicament comprend de la 19-noréthistérone ou de l'acétate de 19-noréthistérone.

6. Matrice selon l'une quelconque des revendications 1 à 4, où le médicament comprend un mélange d'estrogène et de progestatif.

7. Dispositif de délivrance transdermique ou transmuqueux de médicament pour l'administration d'au moins un médicament stéroïdien à une zone de peau ou de muqueuse, comprenant :
a) une couche de matériau support qui est essentiellement imperméable au médicament et qui lui est stratifiée ; et
b) une matrice adhésive contenant un médicament, telle que définie dans l'une quelconque des revendications 1 à 6.

8. Dispositif selon la revendication 7 pour l'utilisation dans un procédé de délivrance d'un médicament à un patient par application de la matrice du dispositif à la peau ou à une muqueuse dudit patient.

## Revendications (Revendications pour l'(les) Etat(s) contractant(s) suivant(s): ES, GR)

1. Procédé pour la production d'une matrice contenant un médicament pour l'utilisation dans un dispositif de délivrance transdermique ou transmuqueux d'un médicament pour l'administration d'au moins un médicament stéroïdien à une zone de peau ou de muqueuse, lequel procédé comprend la dispersion du médicament en une proportion inférieure ou égale à la saturation dans un corps fait d'un copolymère d'acrylate adhésif sensible à la pression, ledit copolymère d'acrylate comprenant un copolymère d'au moins l'acrylate de 2-éthythexyle et l'acétate de vinyle, ladite matrice étant essentiellement dépourvue d'un agent améliorant la pénétration cutanée.

2. Procédé selon la revendication 1, où le copolymère comprend approximativement 72 % en poids d'acrylate de 2-éthylhexyle et approximativement 28 % en poids d'acétate de vinyle (Monsanto GELVA® 737).

3. Procédé selon la revendication 1, où le copolymère comprend approximativement 70 % en poids d'acrylate de 2-éthylhexyle et approximativement 30 % en poids d'acétate de vinyle (Monsanto GELVA® 788).

4. Procédé selon la revendication 1, où le copolymère comprend approximativement 85 % en poids d'acrylate de 2-éthylhexyle, approximativement 10 % en poids d'acrylate de méthyle, approximativement 3 % en poids d'acide acrylique et approximativement 2 % en poids d'acétate de vinyle (Morton Thiokol Morstik 607).

5. Procédé selon l'une quelconque des revendications 1 à 4, où le médicament comprend de la 19-noréthistérone ou de l'acétate de 19-noréthistérone.

6. Procédé selon l'une quelconque des revendications 1 à 4, ou le médicament comprend un mélange d'estrogène et de progestatif.

7. Procédé pour production d'un dispositif de délivrance transdermique ou transmuqueux de médicament pour l'administration d'au moins un médicament stéroïdien à une zone de peau ou de muqueuse, lequel procédé comprend la stratification :
a) d'une couche de matériau support qui est essentiellement imperméable au médicament ; et
b) d'une matrice adhésive contenant un médicament, pourvant être obtenue selon un procédé tel que défini dans l'une quelconque des revendications 1 à 6.
